# EUROPÄISCHE PATENTSCHRIFT

(11) **EP 0 703 226 B1**
(45) Veröffentlichungstag und Bekanntmachung des Hinweises auf die Patenterteilung: **03.12.1997**
(21) Anmeldenummer: 95114215.7
(22) Anmeldetag: 11.09.1995
(51) Int. Cl.: C07D 249/12, C07C 281/06

(54) **Verfahren zur Herstellung von Alkoxytriazolinonen**
Process for the preparation of alcoxytriazolinones
Procédé pour la préparation d'alcoxytriazolinones

(30) Priorität: 23.09.1994 DE 4433966
(43) Veröffentlichungstag der Anmeldung: 27.03.1996
(73) Patentinhaber: BAYER AG, 51368 Leverkusen (DE)
(72) Erfinder: Wroblowsky, Heinz-Jürgen, Dr., D-40764 Langenfeld (DE); König, Klaus, Dr., D-51519 Odenthal (DE)

(56) Entgegenhaltungen:
- EP-A- 0 477 646
- EP-A- 0 507 171

## Beschreibung

Die Erfindung betrifft ein neues, auch technisch durchführbares Verfahren zur Herstellung von weitgehend bekannten Alkoxytriazolinonen, welche als Zwischenprodukte zur Herstellung von agrochemischen Wirkstoffen verwendet werden können.

Alkoxytriazolinone und mehrere Methoden zu ihrer Herstellung sind bereits bekannt (vgl. J. Indian Chem. Soc. 6 (1929), 565-575; J. Chem. Soc. Perkin I 1973, 2644-2646; Arch. Pharm. 307 (1974), 889-891; EP-A 477646; EP-A 507171). Nach diesen bekannten Synthesemethoden werden Alkoxytriazolinone jedoch nur in sehr unbefriedigenden Ausbeuten erhalten.

Ferner ist bekannt, daß durch Methylierung von Urazol oder 4-Methylurazol mit Diazomethan (CH₂N₂) 5-Methoxy-4-methyl-2,4-dihydro-3H-1,2,4-triazol-3-on gebildet wird [vergl. F. Arndt et al, Rev. Fac. Sci. Istanbul 13A, S. 127 bis 144 (1948)]; diese Methode liefert zwar das Triazolinon in hoher Ausbeute, kann aber technisch nicht durchgeführt werden.

Es wurde nun gefunden, daß man Alkoxytriazolinone der allgemeinen Formel (I) 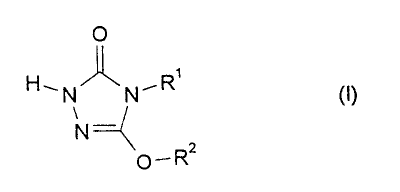 in welcher
- R¹: für Wasserstoff oder für jeweils gegebenenfalls substituiertes Alkyl, Alkenyl, Alkinyl, Cycloalkyl, Cycloalkylalkyl, Aryl oder Arylalkyl steht und
- R²: für jeweils gegebenenfalls substituiertes Alkyl, Alkenyl, Alkinyl, Cycloalkyl, Cycloalkylalkyl, Aryl oder Arylalkyl steht,
in sehr guten Ausbeuten und in hoher Reinheit erhält, wenn man Imino(thio)kohlensäurediester der allgemeinen Formel (II) 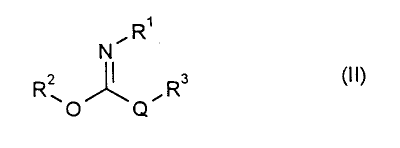 in welcher
- R¹ und R²: die oben angegebenen Bedeutungen haben,
- Q: für Sauerstoff oder Schwefel steht und
- R³: für jeweils gegebenenfalls substituiertes Alkyl, Aryl oder Arylalkyl steht,
mit Carbazinsäure-alkoxyethylestern der allgemeinen Formel (III) 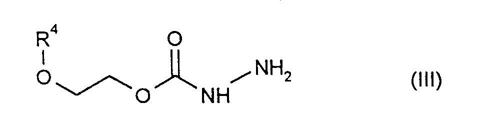 in welcher
- R⁴: für gegebenenfalls substituiertes Alkyl steht,
gegebenenfalls in Gegenwart eines Reaktionshilfsmittels und gegebenenfalls in Gegenwart eines Verdünnungsmittels bei Temperaturen zwischen -20°C und +150°C umsetzt ("erste Umsetzungsstufe") und die hierbei gebildeten Semicarbazid-Derivate der allgemeinen Formel (IV) 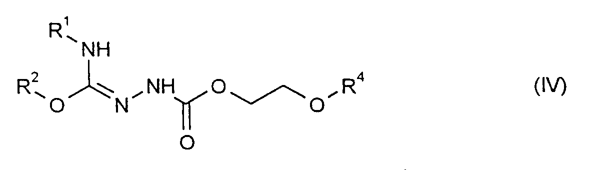 in welcher
- R¹, R² und R⁴: die oben angegebenen Bedeutungen haben,
- und/oder die entsprechenden hierzu tautomeren Verbindungen -
gegebenenfalls nach Zwischenisolierung gegebenenfalls in Gegenwart eines Verdünnungsmittels bei Temperaturen zwischen 0°C und 200°C, gegebenenfalls unter vermindertem Druck, cyclisierend kondensiert ("zweite Umsetzungsstufe").

Überraschenderweise können die Alkoxytriazolinone der allgemeinen Formel (I) nach dem erfindungsgemäßen Verfahren in erheblich höheren Ausbeuten als nach den meisten bekannten Synthesemethoden erhalten werden.

Gegenüber der "Diazomethan-Methode" (F. Arndt et al., l.c.) hat das erfindungsgemäße Verfahren den entscheidenden Vorteil, daß es auch technisch durchführbar ist.

Im Gegensatz zum bekannten Verfahren, bei welchem Phenol als Koppelprodukt entsteht - R⁴ steht für Phenyl (vgl. EP-A 507171, Beispiele II-1 und II-2) - kann das erfindungsgemäße Verfahren in der zweiten Stufe problemlos - ohne Zusatz einer Base - unter Abspaltung von Alkoxyethanolen durchgeführt werden, welche wesentlich einfacher und mit geringerem Aufwand als Phenol zurückgewonnen werden können.

Ein besonderer Vorteil des erfindungsgemäßen Verfahrens besteht darin, daß die zu verwendenden Carbazinsäureester der Formel (III) vielfach bei Raumtemperatur und bei der Umsetzungstemperatur flüssig sind und somit auf die Verwendung eines Verdünnungsmittels in vielen Fällen problemlos verzichtet werden kann.

Das erfindungsgemäße Verfahren stellt somit eine wertvolle Bereicherung des Standes der Technik dar.

Die Erfindung betrifft vorzugsweise die Herstellung von Verbindungen der Formel (I), in welcher
- R¹: für Wasserstoff oder für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder (C₁-C₄-Alkoxy)-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht und
- R²: für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder (C₁-C₄-Alkoxy)-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht.

Die Erfindung betrifft insbesondere die Herstellung von Verbindungen der Formel (I), in welcher
- R¹: für jeweils gegebenenfalls durch Fluor, Chlor, Brom, Methoxy oder Ethoxy substituiertes Methyl, Ethyl, n- oder i-Propyl, n-, i-, s- oder t-Butyl, für jeweils gegebenenfalls durch Fluor und/oder Chlor substituiertes Propenyl, Butenyl, Propinyl oder Butinyl, für jeweils gegebenenfalls durch Fluor, Chlor, Methyl oder Ethyl substituiertes Cyclopropyl oder Cyclopropylmethyl, oder für jeweils gegebenenfalls durch Fluor, Chlor, Brom, Methyl, Ethyl, Trifluormethyl, Methoxy, Ethoxy, Difluormethoxy oder Trifluormethoxy substituiertes Phenyl oder Benzyl steht und
- R²: für jeweils gegebenenfalls durch Fluor, Chlor, Brom, Methoxy oder Ethoxy substituiertes Methyl, Ethyl, n- oder i-Propyl, n-, i-, s- oder t-Butyl, für jeweils gegebenenfalls durch Fluor und/oder Chlor substituiertes Propenyl, Butenyl, Propinyl oder Butinyl, für jeweils gegebenenfalls durch Fluor, Chlor, Brom, Methyl oder Ethyl substituiertes Cyclopropyl oder Cyclopropylmethyl, oder für jeweils gegebenenfalls durch Fluor, Chlor, Brom, Methyl, Ethyl, Trifluormethyl, Methoxy, Ethoxy, Difluormethoxy oder Trifluormethoxy substituiertes Phenyl oder Benzyl steht.

Verwendet man beispielsweise Methylimino-kohlensäure-dimethylester und Carbazinsäure-(2-methoxyethyl)-ester als Ausgangsstoffe, so kann der Reaktionsablauf beim erfindungsgemäßen Verfahren durch das folgende Formelschema skizziert werden:

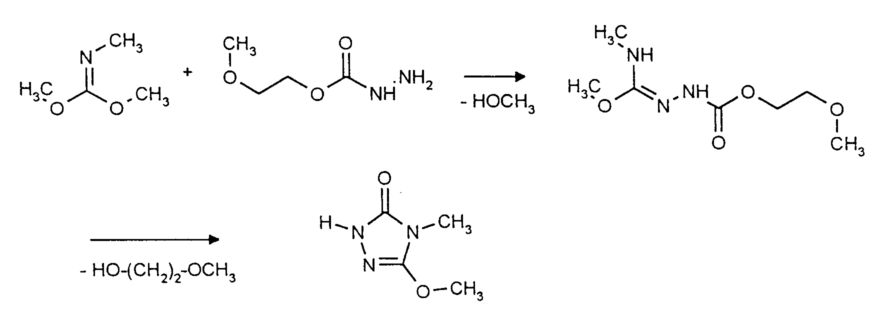

Die beim erfindungsgemäßen Verfahren zur Herstellung der Verbindungen der allgemeinen Formel (I) als Ausgangsstoffe zu verwendenden Imino(thio)kohlensäurediester sind durch die Formel (II) allgemein definiert. In der Formel (II) haben R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹ und R² angegeben wurden; R³ steht vorzugsweise für gegebenenfalls durch Hydroxy, Carboxy, C₁-C₄-Alkoxy, C₁-C₄-Alkoxy-carbonyl, Phenoxy oder Benzyloxy substituiertes C₁-C₄-Alkyl, Phenyl oder Benzyl, insbesondere für Methyl, Ethyl, Methoxyethyl oder Ethoxyethyl.

Die Ausgangsstoffe der Formel (II) sind bekannt und/oder können nach an sich bekannten Verfahren hergestellt werden (vgl. Chem. Ber. 120 (1987), 339-344; Herstellungsbeispiele).

Die beim erfindungsgemäßen Verfahren weiter als Ausgangsstoffe zu verwendenden Carbazinsäure-alkoxyethylester sind durch die Formel (III) allgemein definiert. In der Formel (III) steht R⁴ vorzugsweise für gegebenenfalls durch C₁-C₄-Alkoxy, Phenoxy oder Benzyloxy substituiertes C₁-C₄-Alkyl, insbesondere für Methyl, Ethyl, 2-Methoxyethyl oder 2-Ethoxyethyl.

Die Ausgangsstoffe der Formel (III) sind bekannt und/oder können nach an sich bekannten Verfahren hergestellt werden (vgl. Chem. Ber. 114 (1981), 2001-2018; Herstellungsbeispiele).

Die beim erfindungsgemäßen Verfahren zur Herstellung der Verbindungen der allgemeinen Formel (I) als Zwischenprodukte anfallenden Semicarbazid-Derivate sind durch die Formel (IV) allgemein definiert. In der Formel (IV) haben R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹ und R² angegeben wurden; R⁴ steht vorzugsweise für gegebenenfalls durch C₁-C₄-Alkoxy, Phenoxy oder Benzyloxy substituiertes C₁-C₄-Alkyl, insbesondere für Methyl, Ethyl, 2-Methoxyethyl oder 2-Ethoxyethyl.

Die Zwischenprodukte der Formel (IV) sind noch nicht aus der Literatur bekannt; sie sind als neue Stoffe auch Gegenstand der vorliegenden Anmeldung.

Als Verdünnungsmittel zur Durchführung des erfindungsgemäßen Verfahrens kommen (in beiden Umsetzungsstufen) die üblichen organischen Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Das erfindungsgemäße Verfahren kann jedoch mit gutem Erfolg auch ohne Verwendung eines der oben angegebenen Verdünnungsmittel durchgeführt werden.

Das erfindungsgemäße Verfahren wird in der ersten Stufe vorzugsweise in Gegenwart eines geeigneten Reaktionshilfsmittels durchgeführt. Als solche kommen vorzugsweise Protonensäuren, wie z.B. Salzsäure, Schwefelsäure, Phosphorsäure, Kohlensäure, Essigsäure, Propionsäure, Pivalinsäure, Methansulfonsäure, Benzoesäure, Benzolsulfonsäure und p-Toluolsulfonsäure, gegebenenfalls aber auch saure Ionenaustauscher in Betracht.

Pivalinsäure, (wäßrige) Salzsäure und Essigsäure werden als Reaktionshilfsmittel bei der ersten Stufe des erfindungsgemäßen Verfahrens besonders bevorzugt.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen -20°C und +150°C, vorzugsweise bei Temperaturen zwischen 0°C und 120°C, insbesondere bei Temperaturen zwischen 10°C und 100°C.
Die Reaktionstemperaturen können bei der Durchführung der zweiten Stufe des erfindungsgemäßen Verfahrens in einem größeren Bereich variiert werden. Im allgemeinen arbeitet man bei Temperaturen zwischen 0°C und 200°C, vorzugsweise bei Temperaturen zwischen 50°C und 180°C.

Das erfindungsgemäße Verfahren wird in der ersten Stufe im allgemeinen unter Normaldruck durchgeführt. Es ist jedoch auch möglich, unter erhöhtem oder vermindertem Druck - im allgemeinen zwischen 0,1 bar und 10 bar - zu arbeiten.

In der zweiten Stufe wird das erfindungsgemäße Verfahren im allgemeinen unter vermindertem Druck - vorzugsweise zwischen 0,001 und 100 mbar, insbesondere zwischen 0,01 und 50 mbar - durchgeführt.

Zur Durchführung des erfindungsgemäßen Verfahrens zur Herstellung der Verbindungen der Formel (I) setzt man pro Mol Imino(thio)kohlensäurediester der Formel (II) im allgemeinen 0,5 bis 1,2 Mol, vorzugsweise 0,8 bis 1,1 Mol Carbazinsäure-alkoxyethylester der Formel (III) und gegebenenfalls 0,001 bis 2,0 Mol, vorzugsweise 0,01 bis 1,0 Mol Reaktionshilfsmittel ein.

In einer bevorzugten Ausführungsform des erfindunggemäßen Verfahrens werden die Ausgangsstoffe der Formel (II) und der Formel (III) sowie gegebenenfalls ein Reaktionshilfsmittel - gegebenenfalls in einem geeigneten Verdünnungsmittel - vermischt und bei der erforderlichen Temperatur gerührt, bis praktisch kein Ausgangsmaterial mehr vorhanden ist. Die Isolierung des Zwischenproduktes der Formel (IV) kann dann auf übliche Weise durchgeführt werden. Das Zwischenprodukt der Formel (IV) kann aber auch ohne Zwischenisolierung - gegebenenfalls in einem der oben angegebenen Verdünnungsmittel gelöst - bei der zur cyclisierenden Kondensation erforderlichen Temperatur bis zum Ende der Umsetzung gerührt und anschließend durch Destillation - vorzugsweise unter vermindertem Druck - isoliert werden.

Die nach dem erfindungsgemäßen Verfahren herzustellenden Verbindungen der Formel (I) können als Zwischenprodukte zur Herstellung von herbizid wirksamen Verbindungen verwendet werden (vgl. EP-A 477646 und EP-A 507171).

### Herstellungsbeispiele:

### Beispiel 1

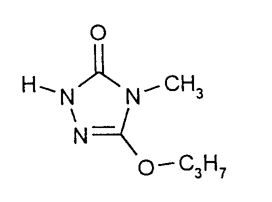

Eine Mischung aus 888 g (6,0 Mol) Carbazinsäure-(2-ethoxyethyl)ester und 954 g (6,0 Mol) Methyliminokohlensäure-di-n-propylester wird 17 Stunden auf 80°C und weitere 2 Stunden auf 100°C erhitzt. Dann werden bei abnehmendem Druck (zuletzt 15 mbar) zunächst Propanol und bereits abgespaltenes Ethoxyethanol abdestilliert, wobei die weitere Umsetzung durch Erhöhen der Temperatur auf 120°C beschleunigt wird. Bei Abklingen der Destillation unter diesen Bedingungen wird bei Normaldruck eine neue -mit Eis gekühlte - Vorlage zum Auffangen des Destillats angesetzt, das Kühlwasser des Kondensatkühlers auf 80°C aufgeheizt und die weitere Destillation im Ölpumpenvakuum durchgeführt. Die Sumpftemperatur wird dann allmählich auf 150°C bis 170°C erhöht. Im Verlauf von 2 Stunden werden 823 g eines leicht gelblichen Destillats erhalten, welches allmählich kristallin erstarrt und nach GC-Analyse 87,2% 4-Methyl-5-propoxy-2,4-dihydro-3H-1,2,4-triazol-3-on enthält. Ausbeute: 76% der Theorie.

Durch erneute Destillation wird ein 98%iges Produkt vom Schmelzpunkt 67°C erhalten.

### Beispiel 2

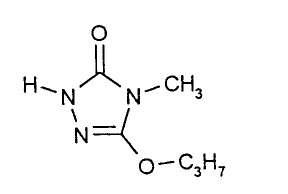

Eine Mischung aus 296 g (2,0 Mol) Carbazinsäure-(2-ethoxyethyl)ester und 318 g (2,0 Mol) Methyliminokohlensäure-di-n-propylester wird bei ca. 10°C mit einer Lösung von 1,0 g (0,01 Mol) Pivalinsäure in 1,5 ml n-Propanol versetzt und die Mischung wird 4 Stunden bei 10°C bis 20°C gerührt. Nach Zugabe von zusätzlich 1,0 g Pivalinsäure wird das Gemisch noch 12 Stunden bei 20°C bis 25°C gerührt. Anschließend wird bei ca. 100°C Sumpftemperatur und 15 mbar Druck Propanol abdestilliert. Dann werden bei ca. 140°C Sumpftemperatur und 15 mbar Druck innerhalb von ca. 90 Minuten ca. 170 g Ethoxyethanol abdestilliert. Bei Abklingen der Destillation unter diesen Bedingungen wird bei Normaldruck eine neue - mit Eis gekühlte - Vorlage zum Auffangen des Destillats angesetzt, das Kühlwasser des Kondensatkühlers auf 80°C aufgeheizt und die weitere Destillation im Ölpumpenvakuum durchgeführt. Die Sumpftemperatur wird dann allmählich auf 140°C bis 180°C erhöht; die Kopftemperatur beträgt ca. 135°C, der Druck ca. 1 mbar.

Man erhält 275 g eines Destillates, welches nach GC-Analyse 95,3% 4-Methyl-5-propoxy-2,4-dihydro-3H-1,2,4-triazol-3-on enthält. Ausbeute: 83,5% der Theorie.

### Beispiel 3

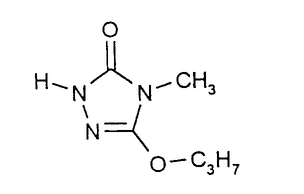

In eine Mischung aus 37,7 g (0,239 Mol) Carbazinsäure-(2-ethoxyethyl)ester, 38 g (0,239 Mol) Methylimino-thiokohlensäure-O-n-propylester-S-methylester, 50 ml n-Propanol und 4,5 g Wasser wird bei 40°C 12 Stunden lang Kohlendioxid eingeleitet. Durch Eindampfen und Destillation unter Vakuum (Ölpumpe) erhält man 40 g eines Rohdestillats, das zu 66,1 % das gewünschte 4-Methyl-5-propoxy-2,4-dihydro-3H-1,2,4-triazol-3-on enthält. Ausbeute: 67,4 % der Theorie.

### Beispiel 4

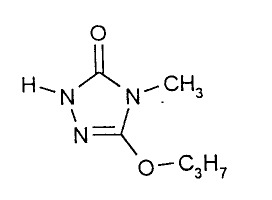

Eine Mischung auis 37,7 g (0,239 Mol) Carbazinsäure-(2-ethoxyethyl)ester, 38 g (0,239 Mol) Methyliminothiokohlensäure-O-n-propylester-S-methylester und 50 ml n-Propanol wird bei 0°C mit einem Äquivalent Pivalinsäure versetzt. Man läßt 2 Stunden bei Raumtemperatur nachrühren, engt im Wasserstrahlpumpenvakuum ein und destilliert anschließend im Ölpumpenvakuum. Man erhält 39 g eines Rohdestillats, das zu 80,6 % aus dem gewünschten 4-Methyl-5-propoxy-2,4-dihydro-3H-1,2,4-triazol-3-on besteht. Ausbeute: 80,1 % der Theorie.

### Beispiel 5

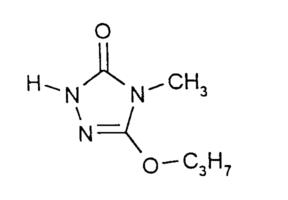

74 g (0,5 Mol) Carbazinsäure-(2-ethoxyethyl)ester werden in 150 ml n-Propanol vorgelegt und unter Eiskühlung mit einer Lösung von 18,3 g (0,5 Mol) Chlorwasserstoffgas in 98 g n-Propanol versetzt. Dann werden unter weiterer Eiskühlung 95,5 g (0,5 Mol) Methyliminothiokohlensäure-O-n-propylester-S-(2-methoxyethyl)ester innerhalb von 30 Minuten zugetropft. Nach einer weiteren Stunde bei 20°C wird durch Zugabe von 0,5 Mol 30 %iger methanolischer Natriummethanolat-Lösung neutralisiert und dann im Vakuum eingeengt. Ab 120°C/1 mbar destilliert man das Produkt vom gebildeten Kochsalz ab, wobei 53,1 g eines Rohdestillats erhalten werden, das zu 92,2 % das gewünschte Triazolinon der obigen Formel enthält. Ausbeute: 62,4 % der Theorie.

### Beispiel 6

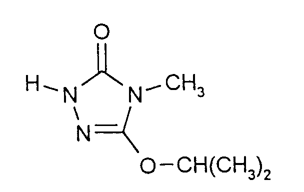

Ein Gemisch aus 148 g (1 Mol) Methyliminothiokohlensäure-O-isopropylester-S-methylester und 147 g (1 Mol) Carbazinsäure-(2-ethoxyethyl)ester wird 5 Stunden auf 120°C erhitzt. Danach kommt die Gasentwickiung zum Erliegen. Nun wird bei 20 mbar eingeengt und anschließend bei 1 mbar über eine kurze Kolonne destilliert. Es werden 103 g eines Rohdestillats erhalten, das zu 62,1 % aus dem gewünschten Produkt besteht (Ausbeute: 40,7 % der Theorie). Nach Umkristallisation aus Toluol erhält man das 4-Methyl-5-isopropoxy-2,4-dihydro-3H-1,2,4-triazol-3-on als farblosen Feststoff vom Schmelzpunkt 140 bis 141°C.

### Beispiel 7

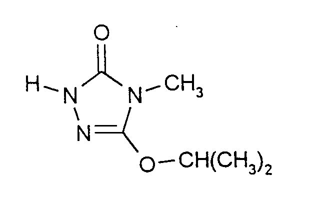

182 g (1,23 Mol) Carbazinsäure-(2-ethoxyethyl)ester werden mit 161 g (1,23 Mol) Methyliminokohlensäure-O-methylester-O-i-propylester vermischt und nach Zugabe von 3 g Pivalinsäure wird die Mischung 8 Stunden bei 20°C und eine weitere Stunde bei 40°C gerührt. Dann wird im Wasserstrahlvakuum bei einer Sumpftemperatur von maximal 100°C eingeengt und dann im Ölpumpenvakuum bei einer Sumpftemperatur von maximal 180°C destilliert. Das Destillat wird aus Toluol umkristallisiert.

Man erhält 164 g (85% der Theorie) 4-Methyl-5-i-propoxy-2,4-dihydro-3H-1,2,4-triazol-3-on vom Schmelzpunkt 142°C.

### Beispiel 8

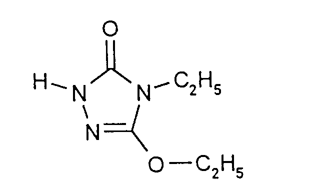

Analog zu Beispiel 2 erhält man durch Umsetzung von äquimolaren Mengen von Carbazinsäure-(2-ethoxyethyl)-ester und Ethylimino-kohlensäure-0,0-diethylester in Gegenwart von Pivalinsäure (2 Mol-%) und cyclisierende Kondensation des gebildeten Zwischenproduktes 5-Ethoxy-4-ethyl-2,4-dihydro-3H-1,2,4-triazol-3-on (Ausbeute: 85 % d. Th. nach Destillation) vom Schmelzpunkt 115°C (aus Toluol).

### Beispiel 9

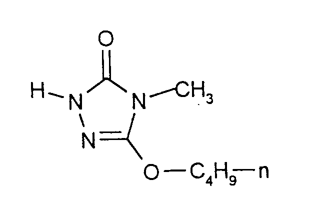

Analog zu den Beispielen 2 und 8 erhält man 5-n-Butoxy-4-methyl-2,4-dihydro-3H-1,2,4-triazol-3-on (Ausbeute: 76 % d. Th.) vom Schmelzpunkt 77°C (aus Cyclohexan/Toluol 4:1 (v/v)).

### Ausgangsstoffe der Formel (III):

### Beispiel (III-1)

H₂N-NH-CO-O-CH₂CH₂-O-C₂H₅

1125 g (22,5 Mol) Hydrazinhydrat werden im Verlauf von 2 Stunden tropfenweise zu 4635 g (22,5 Mol) Bis-(2-ethoxyethyl)-carbonat gegeben und die Mischung wird dann 2 Stunden bei 60°C und eine weitere Stunde bei 80°C gerührt. Dann werden bei 15 mbar Wasser und Ethoxyethanol abdestilliert und das verbleibende Rohprodukt wird durch Destillation im Ölpumpenvakuum gereinigt.

Man erhält 3164 g (95% der Theorie) Carbazinsäure-(2-ethoxyethyl)ester vom Siedepunkt 99°C (bei 0,15 mbar).

Analog können beispielsweise auch Carbazinsäure-(2-methoxyethyl)ester, Carbazinsäure-(2-propoxyethyl)ester, Carbazinsäure-(2-butoxyethyl)ester, Carbazinsäure-[2-(2-methoxyethoxy)]ethylester, Carbazinsäure[2-(2-ethoxyethoxy)]ethylester und Carbazinsäure-(2-benzyloxyethyl)ester hergestellt werden.

## Patentansprüche

1. Verfahren zur Herstellung von Alkoxytriazolinonen der allgemeinen Formel (I), 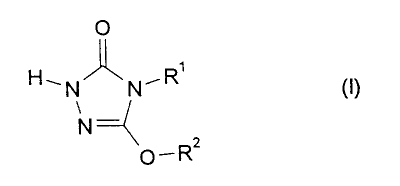 in welcher
R¹ für Wasserstoff oder für jeweils gegebenenfalls substituiertes Alkyl, Alkenyl, Alkinyl, Cycloalkyl, Cycloalkylalkyl, Aryl oder Arylalkyl steht und
R² für jeweils gegebenenfalls substituiertes Alkyl, Alkenyl, Alkinyl, Cycloalkyl, Cycloalkylalkyl, Aryl oder Arylalkyl steht,
dadurch gekennzeichnet, daß man Imino(thio)kohlensäurediester der allgemeinen Formel (II), 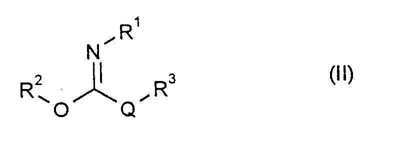 in welcher
R¹ und R² die oben angegebenen Bedeutungen haben,
Q für Sauerstoff oder Schwefel steht und
R³ für jeweils gegebenenfalls substituiertes Alkyl, Aryl oder Arylalkyl steht,
mit Carbazinsäure-alkoxyethylestern der allgemeinen Formel (III), 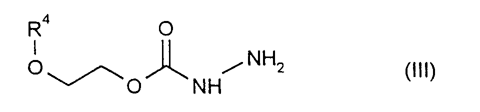 in welcher
R⁴ für gegebenenfalls substituiertes Alkyl steht,
gegebenenfalls in Gegenwart eines Reaktionshilfsmittels und gegebenenfalls in Gegenwart eines Verdünnungsmittels bei Temperaturen zwischen -20°C und +150°C umsetzt ("erste Umsetzungsstufe") und die hierbei gebildeten Semicarbazid-Derivate der allgemeinen Formel (IV) 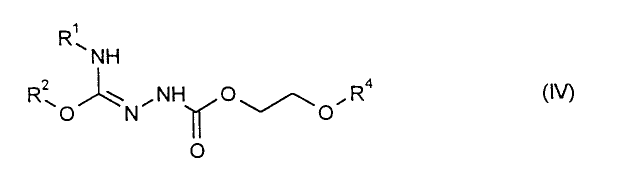 in welcher
R¹, R² und R⁴ die oben angegebenen Bedeutungen haben,
- und/oder die entsprechenden hierzu tautomeren Verbindungen -
gegebenenfalls nach Zwischenisolierung, gegebenenfalls in Gegenwart eines Verdünnungsmittels bei Temperaturen zwischen 0°C und 200°C, gegebenenfalls unter vermindertem Druck, cyclisierend kondensiert ("zweite Umsetzungsstufe").

2. Verfahren nach Anspruch 1, dadurch gekennzeichnet, daß Verbindungen der Formel (I) hergestellt werden, in welcher
R¹ für Wasserstoff oder für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder (C₁-C₄-Alkoxy)-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht und
R² für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder (C₁-C₄-Alkoxy)-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht.

3. Verfahren nach Anspruch 1, dadurch gekennzeichnet, daß man die erste Umsetzungsstufe bei Temperaturen zwischen 0°C und 120°C, insbesondere zwischen 10°C und 100°C durchführt.

4. Verfahren nach Anspruch 1, dadurch gekennzeichnet, daß man die zweite Umsetzungsstufe bei Temperaturen zwischen 50°C und 180°C durchführt.

5. Verfahren nach Anspruch 1, dadurch gekennzeichnet, daß man die zweite Umsetzungsstufe unter vermindertem Druck, vorzugsweise zwischen 0,001 und 100 mbar, insbesondere zwischen 0,01 und 50 mbar, durchführt.

6. Verfahren nach Anspruch 1, dadurch gekennzeichnet, daß als Imino(thio)kohlensäureester der Formel (II) Methyliminokohlensäure-di-n-propylester, Methyliminokohlensäure-O-methylester-O-isopropylester, Methyliminothiokohlensäure-O-n-propylester-S-methylester, Methyliminothiokohlensäure-O-isopropylester-S-methylester oder Methyliminothiokohlensäure-O-n-propylester-S-(2-methoxyethyl)ester eingesetzt wird.

7. Verfahren nach Anspruch 1, dadurch gekennzeichnet, daß als Carbazinsäure-alkoxyalkylester der Formel (III) Carbazinsäure-(2-ethoxyethyl)ester eingesetzt wird.

8. Verfahren nach Anspruch 1, dadurch gekennzeichnet, daß in der ersten Umsetzungsstufe als Reaktionshilfsmittel eine Protonensäure, insbesondere Pivalinsäure, (wäßrige) Salzsäure oder Essigsäure eingesetzt wird.

9. Semicarbazid-Derivate der Formel (IV) 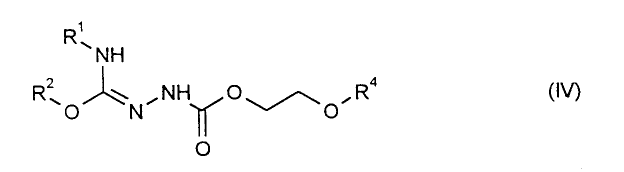 in welcher
R¹ für Wasserstoff oder für jeweils gegebenenfalls substituiertes Alkyl, Alkenyl, Alkinyl, Cycloalkyl, Cycloalkylalkyl, Aryl oder Arylalkyl steht,
R² für jeweils gegebenenfalls substituiertes Alkyl, Alkenyl, Alkinyl, Cycloalkyl, Cycloalkylalkyl, Aryl oder Arylalkyl steht und
R⁴ für gegebenenfalls substituiertes Alkyl steht,
sowie die hiermit tautomeren Verbindungen.

## Claims

1. Process for the preparation of alkoxytriazolinones of the general formula (I), 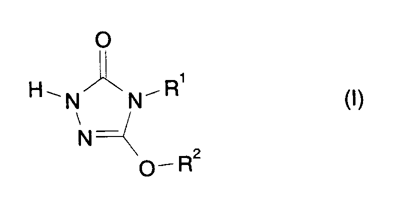 in which
R¹ represents hydrogen or represents in each case optionally substituted alkyl, alkenyl, alkinyl, cycloalkyl, cycloalkylalkyl, aryl or arylalkyl and
R² represents in each case optionally substituted alkyl, alkenyl, alkinyl, cycloalkyl, cycloalkylalkyl aryl or arylalkyl,
characterized in that imino(thio)carbonic diesters of the general formula (II), 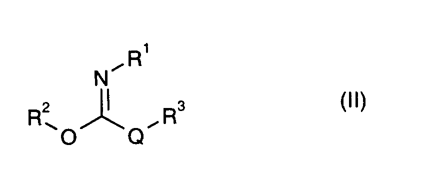 in which
R¹ and R² have the abovementioned meanings,
Q represents oxygen or sulphur and
R³ represents in each case optionally substituted alkyl, aryl or arylalkyl,
are reacted with alkoxyethyl carbazinates of the general formula (III), 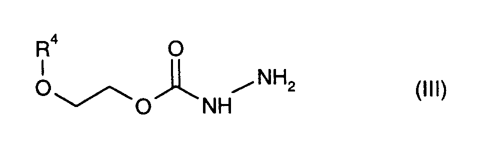 in which
R⁴ represents optionally substituted alkyl,
if appropriate in the presence of a reaction auxiliary and if appropriate in the presence of a diluent at temperatures between -20°C and +150°C ("first reaction step") and the semicarbazide derivatives formed in this process of the general formula (IV) 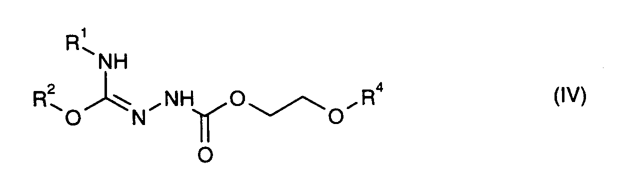 in which
R¹, R² and R⁴ have the abovementioned meanings,
- and/or the corresponding tautomeric compounds -
are subjected to a cyclizing condensation reaction, at temperatures between 0°C and 200°C, if appropriate after intermediate isolation, and if appropriate in the presence of a diluent, if appropriate under reduced pressure ("second reaction step").

2. Process according to Claim 1, characterized in that compounds of the formula (I) are prepared in which
R¹ represents hydrogen, or represents alkyl, alkenyl or alkinyl, each of which has up to 6 carbon atoms and each of which is optionally substituted by halogen or C₁-C₄-alkoxy, or represents cycloalkyl or cycloalkylalkyl, each of which has 3 to 6 carbon atoms in the cycloalkyl moiety and, if appropriate, 1 to 4 carbon atoms in the alkyl moiety and each of which is optionally substituted by halogen or C₁-C₄-alkyl, or represents aryl or arylalkyl, each of which has 6 or 10 carbon atoms in the aryl moiety and, if appropriate, 1 to 4 carbon atoms in the alkyl moiety and each of which is optionally substituted by carboxyl, cyano, nitro, halogen, C₁-C₄-alkyl, C₁-C₄-halogenoalkyl, C₁-C₄-alkoxy, C₁-C₄-halogenoalkoxy or (C₁-C₄-alkoxy)-carbonyl, and
R² represents alkyl, alkenyl or alkinyl, each of which has up to 6 carbon atoms and each of which is optionally substituted by halogen or C₁-C₄-alkoxy, or represents cycloalkyl or cycloalkylalkyl, each of which has 3 to 6 carbon atoms in the cycloalkyl moiety and, if appropriate, 1 to 4 carbon atoms in the alkyl moiety and each of which is optionally substituted by halogen or C₁-C₄-alkyl, or represents aryl or arylalkyl, each of which has 6 or 10 carbon atoms in the aryl moiety and, if appropriate, 1 to 4 carbon atoms in the alkyl moiety and each of which is optionally substituted by carboxyl, cyano, nitro, halogen, C₁-C₄-alkyl, C₁-C₄-halogenoalkyl, C₁-C₄-alkoxy, C₁-C₄-halogenoalkoxy or (C₁-C₄-alkoxy)-carbonyl.

3. Process according to Claim 1, characterized in that the first reaction step is carried out at temperatures between 0°C and 120°C, in particular between 10°C and 100°C.

4. Process according to Claim 1, characterized in that the second reaction step is carried out at temperatures between 50°C and 180°C.

5. Process according to Claim 1, characterized in that the second reaction step is carried out under reduced pressure, preferably between 0.001 and 100 mbar, in particular between 0.01 and 50 mbar.

6. Process according to Claim 1, characterized in that the imino(thio)carbonic ester of the formula (II) employed is di-n-propyl methyliminocarbonate, O-methyl O-isopropyl methyliminocarbonate, O-n-propyl S-methyl methyliminothiocarbonate, O-isopropyl S-methyl methyliminothiocarbonate or O-n-propyl S-(2-methoxyethyl) methyliminothiocarbonate.

7. Process according to Claim 1, characterized in that the alkoxyalkyl carbazinate of the formula (III) employed is 2-ethoxyethyl carbazinate.

8. Process according to Claim 1, characterized in that the reaction auxiliary employed in the first reaction step is a protonic acid, in particular pivalic acid, (aqueous) hydrochloric acid or acetic acid .

9. Semicarbazide derivatives of the formula (IV) 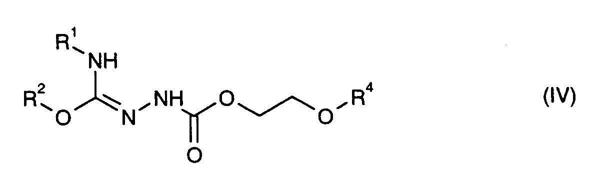 in which
R¹ represents halogen, or represents in each case optionally substituted alkyl, alkenyl, alkinyl, cycloalkyl, cycloalkylalkyl, aryl or arylalkyl,
R² represents in each case optionally substituted alkyl, alkenyl, alkinyl, cycloalkyl, cycloalkylalkyl, aryl or arylalkyl and
R⁴ represents optionally substituted alkyl,
and the tautomeric compounds thereof.

## Revendications

1. Procédé de production d'alkoxytriazolinones de formule générale (I), 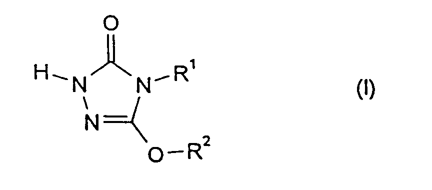 dans laquelle
R¹ représente de l'hydrogène ou un groupe alkyle, alcényle, alcynyle, cycloalkyle, cycloalkylalkyle, aryle ou arylalkyle dont chacun est éventuellement substitué et
R² représente un groupe alkyle, alcényle, alcynyle, cycloalkyle, cycloalkylalkyle, aryle ou arylalkyle dont chacun est éventuellement substitué,
caractérisé en ce qu'on fait réagir des diesters d'acides imino(thio)carboniques de formule générale (II) 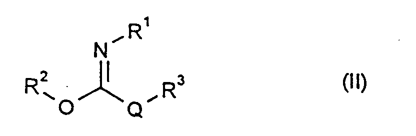 dans laquelle
R¹ et R² ont les définitions indiquées ci-dessus,
Q représente de l'oxygène ou du soufre et
R³ est un groupe alkyle, aryle ou arylalkyle dont chacun est éventuellement substitué,
avec des esters alkoxyéthyliques d'acide carbazique de formule générale (III) 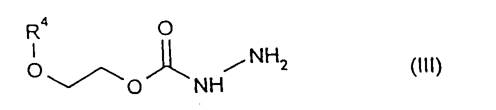 dans laquelle
R⁴ est un groupe alkyle éventuellement substitué, le cas échéant en présence d'un auxiliaire de réaction et en la présence éventuelle d'un diluant, à des températures comprises entre -20°C et +150°C ("premier stade de réaction") et on condense par cyclisation ("second stade de réaction") les dérivés de semicarbazide ainsi formés, de formule générale (IV) 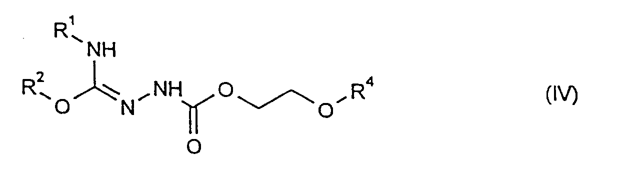 dans laquelle
R¹, R² et R⁴ ont les définitions indiquées ci-dessus,
- et/ou les composés tautomères qui y correspondent -
le cas échéant après isolement intermédiaire, éventuellement en présence d'un diluant à des températures comprises entre 0°C et 200°C, le cas échéant sous pression réduite.

2. Procédé suivant la reendication 1, caractérisé en ce que les composés produits répondent à la formule (I) dans laquelle
R¹ est de l'hydrogène ou un groupe alkyle, alcényle ou alcynyle ayant chacun jusqu'à 6 atomes de carbone et dont chacun est éventuellement substitué par un halogène ou un groupe alkyle en C₁ à C₄, un groupe cycloalkyle ou cycloalkylalkyle ayant chacun 3 à 6 atomes de carbone dans la partie cycloalkyle et, le cas échéant, 1 à 4 atomes de carbone dans la partie alkyle et dont chacun est éventuellement substitué par un halogène ou un groupe alkyle en C₁ à C₄, ou bien un groupe aryle ou arylalkyle ayant chacun 6 ou 10 atomes de carbone dans la partie aryle et, le cas échéant, 1 à 4 atomes de carbone dans la partie alkyle et portant chacun, le cas échéant, un substituant carboxy, cyano, nitro, halogéno, alkyle en C₁ à C₄, halogénalkyle en C₁ à C₄, alkoxy en C₁ à C₄, halogénalxoxy en C₁ à C₄ ou (alkoxy en C₁ à C₄)-carbonyle, et
R² représente un groupe alkyle, alcényle ou alcynyle ayant chacun jusqu'à 6 atomes de carbone et dont chacun est substitué, le cas échéant par un halogène ou un groupe alkoxy en C₁ à C₄, un groupe cycloalkyle ou cycloalkylalkyle ayant chacun 3 à 6 atomes de carbone dans la partie cycloalkyle et, le cas échéant, 1 à 4 atomes de carbone dans la partie alkyle et portant chacun, le cas échéant, un substituant halogéno ou alkyle en C₁ à C₄, ou bien un groupe aryle ou arylalkyle ayant chacun 6 ou 10 atomes de carbone dans la partie aryle et, le cas échéant, 1 à 4 atomes de carbone dans la partie alkyle et portant chacun, le cas échéant, un substituant carboxy, cyano, nitro, halogéno, alkyle en C₁ à C₄, halogénalkyle en C₁ à C₄, alkoxy en C₁ à C₄, halogénalkoxy en C₁ à C₄ ou (alkoxy en C₁ à C₄)-carbonyle.

3. Procédé suivant la revendication 1, caractérisé en ce qu'on conduit le premier stade de réaction à des températures comprises entre 0°C et 120°C, notamment entre 10°C et 100°C.

4. Procédé suivant la revendication 1, caractérisé en ce qu'on conduit le second stade de réaction à des températures comprises entre 50°C et 180°C.

5. Procédé suivant la revendication 1, caractérisé en ce qu'on conduit le second stade de réaction sous pression réduite, de préférence entre 0,001 et 100 mbars, notamment entre 0,01 et 50 mbars.

6. Procédé suivant la revendication 1, caractérisé en ce qu'on utilise comme ester d'acide imino(thio)carbonique de formule (II) l'ester di-n-propylique d'acide méthyliminocarbonique, l'ester de O-méthyle et O-isopropyle d'acide méthyliminocarbonique, l'ester de O-n-propyle et S-méthyle d'acide méthyliminothiocarbonique, l'ester de O-isopropyle et S-méthyle d'acide méthyliminothiocarbonique ou l'ester de O-n-propyle et S-(2-méthoxyéthyle) d'acide méthyliminothiocarbonique.

7. Procédé suivant la revendication 1, caractérisé en ce qu'on utilise comme ester alkoxyalkylique d'acide carbazique de formule (III) l'ester 2-éthoxyéthylique d'acide carbazique.

8. Procédé suivant la revendication 1, caractérisé en ce qu'on utilise comme auxiliaire de réaction dans le premier stade de réaction un acide protonique, notamment l'acide pivalique, l'acide chlorhydrique (aqueux) ou l'acide acétique.

9. Dérivés de semicarbazide de formule (IV) 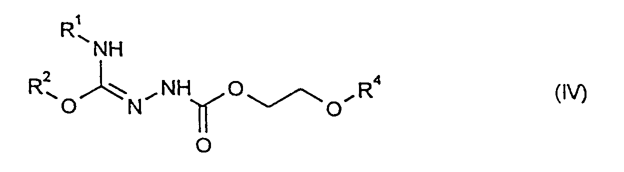 dans laquelle
R¹ représente de l'hydrogène ou un groupe alkyle, alcényle, alcynyle, cycloalkyle, cycloalkylalkyle, aryle ou arylalkyle dont chacun est éventuellement substitué,
R² représente un groupe alkyle, alcényle, alcynyle, cycloalkyle, cycloalkylalkyle, aryle ou arylalkyle dont chacun est éventuellement substitué et
R⁴ est un groupe alkyle éventuellement substitué, ainsi que les composés qui en sont tautomères.
